# EUROPÄISCHE PATENTANMELDUNG

(11) **EP 1 757 316 A2**
(43) Veröffentlichungstag der Anmeldung: **28.02.2007**
(21) Anmeldenummer: 06013714.8
(22) Anmeldetag: 03.07.2006
(51) Int. Cl.: A61L 31/10, A61L 31/16

(54) **Implantat mit immobilisierten Biokatalysatoren**

(30) Priorität: 06.07.2005 DE 102005032691
(71) Anmelder: BIOTRONIK VI Patent AG, 6341 Baar (CH)
(72) Erfinder: Borck, Alexander, Dr., 91086 Aurachtal (DE); Korzuschnik, Ellen, 91077 Neunkirchen am Brand (DE)
(74) Vertreter: Lindner-Vogt, Karin L.

(57) **Zusammenfassung**

Die Erfindung betrifft ein medizinisches Implantat mit an seiner Oberfläche durch kovalente Anbindung immobilisierten Biokatalysatoren.

## Beschreibung

Die Erfindung betrifft ein medizinisches Implantat mit an seiner Oberfläche durch kovalente Anbindung immobilisierten der Biokatalysatoren.

### Hintergrund der Erfindung

Bei medizinischen Implantaten besteht allgemein das Problem, dass Unverträglichkeitsreaktionen auftreten, sei es durch die verwendeten Werkstoffe oder sei es durch die bei und nach der Implantation bzw. bei begleitenden chirurgischen Eingriffen ausgeübten mechanischen Belastungen auf das umgebende Gewebe. Besonders bedeutsam ist dies bei Gefäßstützen (Stents), die zur Verhinderung einer Obstruktion eines zuvor aufgeweiteten Gefäßes implantiert werden. Es besteht die Gefahr eines erneuten Verschlusses des Gefäßes (Restenose), dessen Ursache (i) in einer mechanischen Reizung des umgebenden Gewebes bei und nach der Implantation des Stents (z. B. durch kleine Gefäßverletzungen) oder (ii) in Unverträglichkeitsreaktionen des umgebenden Gewebes mit dem oberflächlich im Implantat verwendeten Material liegen kann.

Unverträglichkeitsreaktionen bei medizinischen Implantaten lassen sich durch den Einsatz neuer biokompatibler Materialien mindern. Um den durch die mechanische Einwirkung induzierten biologischen Prozessen Einhalt zu gebieten, ist eine begleitende systemische Medikation gängig. Neuerdings ist auch die lokale Applikation von Wirkstoffen vorgesehen, die auf die Oberfläche aufgebracht werden und von dort durch Diffusion oder dergleichen allmählich in das umgebende Gewebe freigesetzt werden. Diese Ansätze sind in der Literatur häufig mit dem Begriff ,local drug delivery' und bei Stents auch mit dem Begriff ,drug eluting stent' verknüpft. Die Entwicklung und Auslegung der spezifischen Retardsysteme ist allerdings sehr aufwendig und birgt Risiken. So bestehen technische Schwierigkeiten bezüglich der Festlegung und Einstellung der Höhe und des zeitlichen Verlaufs der Dosierung des Wirkstoffs, der Sicherstellung der Freisetzung des Wirkstoffes an einem gewünschten Ort im Körper und der Auslegung der Prozesse zur Aufbringung der Wirkstoffe auf der Oberfläche des Implantats. Häufig wird es aufgrund der Wirkstoffeigenschaften auch notwendig sein, den Wirkstoff in eine Trägermatrix einzubetten, um eine verfahrenstechnisch sichere Verarbeitung und Aufbringung auf die Stentoberfläche zu ermöglichen und das Retardverhalten im gewünschten Sinne zu beeinflussen. Die Trägermatrix oder ein gegebenenfalls durch Abbau der Trägermatrix entstandenes Addukt kann z. B. jedoch Ausgangspunkt für biologische Prozesse sein, die zum erneuten Verschluss des Gefäßes führen.

### Zusammenfassung der Erfindung

Der vorliegenden Erfindung liegt die Aufgabe zugrunde, die geschilderten Nachteile des Standes der Technik, insbesondere die im Zusammenhang mit einer lokalen Applikation von Wirkstoffen nach Implantation bestehenden Probleme, zu überwinden, zu mindern oder zumindest eine Alternative hierzu bereitzustellen.
Erfindungsgemäß wird diese Aufgabe durch ein medizinisches Implantat mit an seiner Oberfläche durch kovalente Anbindung immobilisierten Biokatalysatoren gelöst.

Der Ansatz unterscheidet sich von dem bisher aus dem Stand der Technik Bekanntem schon dadurch, dass auf die Aufbringung von Wirkstoffen auf die Implantatsoberfläche verzichtet und damit den zuvor geschilderten Problemen bei der Realisation von einen Wirkstoff freisetzenden Implantat aus dem Weg gegangen wird. Stattdessen ist erfindungsgemäß vorgesehen, dass die der Biokatalysatoren kovalent an die Oberfläche des Implantats gebunden werden und sich damit beispielsweise nicht in Blut lösen und ausgespült werden können. Weiterhin werden nur äußerst geringe Mengen dieser Biokatalysatoren benötigt und die eigentlich pharmakologisch wirksamen Addukte der Biokatalysatorenaktivität werden aus den vom Körper bereitgestellten Edukten vor Ort synthetisiert. Eine Wirkstofffreisetzung ist bei Stents und Einsatz von Enzymen zeitlich durch im Blut vorhandene Proteasen terminiert.

Als medizinische Implantate im Sinne der Erfindung werden alle für den permanenten, aber auch zeitlich begrenzten Verbleib im menschlichen Körper vorgesehenen Vorrichtungen angesehen, sei es zu therapeutischen oder diagnostischen Zwecken. Geeignete medizinische Implantate umfassen insbesondere Herzschrittmacher, Defibrillatoren, Herz- und Venenklappen, Gefäßprothesen, aber auch orthopädische Implantate. Vorzugsweise ist das medizinische Implantat ein Stent. Das Implantat besitzt eine äußere Oberfläche, die zur kovalenten Anbindung der Biokatalysatoren entweder aus sich heraus geeignet ist oder erst durch an sich bekannte Behandlungsverfahren zu diesem Zwecke vorbereitet wird. Eine solche Oberfläche wird vorliegend auch als aktivierte Oberfläche bezeichnet.

Als Biokatalysatoren für den erfindungsgemäßen Einsatz kommen alle Biokatalysatoren in Betracht, deren Addukte einen positiven pharmazeutischen Effekt auf das umgebene Gewebe haben. Ein positiver pharmazeutischer Effekt manifestiert sich in einer Verbesserung oder Stabilisierung eines pathologischen Zustands infolge der Freisetzung der bereitgestellten Addukte gegenüber einem hypothetisch ohne die Gegenwart eines solchen Addukts zu konsternierenden pathologischen Zustand. Mit anderen Worten, die Addukte der Biokatalysatorenaktivität Stabilisieren oder Verbessern den pathologischen Zustand oder sie wirken prophylaktisch und mindern das Risiko unerwünschter pathologischer Entwicklungen im Gewebe. Bevorzugt ist der Biokatalysator ein Enzym, kann aber auch ein katalytisch aktiver Antikörper sein. Geeignete Enzyme umfassen insbesondere Katalasen, Peroxidasen, Lipasen und Proteasen.

Bevorzugt ist der Biokatalysator eine Glucoseoxidase (GOD, EC 1.1.3.4). Andere Namen sind Glukoseoxihydrase, Corylophyline, Penantin, Glukoseaerodehydrogenase, Mikrocid, β-D-Glucoseoxidase, D-Glucoseoxidase, D-Glukose-1-oxidase, β-D-Glukose: Quinonoxidoreduktase, Glukoseoxyhydrase und Deoxin-1 und der systematische Name lautet β-D-Glukose:Sauerstoff-1-oxidoreduktase. GOD ist ein homodimeres Enzym. Jede Untereinheit ist mit dem Coenzym Flavin-adenin-dinukleotid (FAD) assoziiert. Jedes GOD-Monomer hat zwei eindeutige Bindungsdomänen: eine Domäne bindet sich an FAD, die andere an das Substrat β-D-Glukose. GOD gehört zu den Flavoenzymen und katalysiert die Oxidation von β-D-Glukose mit molekularem Sauerstoff zu β-D-Glukono-δ-lakton unter Bildung von Wasserstoffperoxid H₂O₂. Das Addukt Wasserstoffperoxid ist zelltoxisch und sollte aus diesem Grunde insbesondere einen die Restenoserate senkenden Effekt nach Stentimplantation besitzen. Daher ist der Einsatz einer Glucoseoxidase erfindungsgemäß bevorzugt. Darüber hinaus eignet sich ein medizinisches Implantat mit GOD zur lokalen Krebsbehandlung.

Bevorzugt ist weiterhin, dass Biokatalysator eine Peroxidase (Unter-Unterklasse EC 1.11.1), insbesondere Katalase (EC 1.11.1.6) ist. Polymorphkernigen neutrophilen Granulozyten (PMN) sind ein wichtiger Bestandteil der unspezifischen Abwehr des Körpers und sind an Entzündungsprozessen im Körper beteiligt. Sie schützen den Organismus vor schädigenden Einflüssen in Form von Mikroorganismen oder anderen Fremdkörpern, können jedoch auch apoptotische, geschädigte, überalterte oder opsonisierte körpereigene Zellen erkennen und beseitigen. Eine der wichtigsten funktionellen Eigenschaften der neutrophilen Granulozyten ist die Bildung reaktiver Sauerstoffspezies, die Wasserstoffperoxid umfassen. Um etwaigen durch die Implantation induzierten entzündlichen Prozessen entgegenzuwirken, werden Peroxidasen, insbesondere Katalase verwendet, die Wasserstoffperoxid abbauen. Daher ist der Einsatz einer Peroxidase, insbesondere von Katalase erfindungsgemäß bevorzugt.

Bevorzugt ist weiterhin, dass der Biokatalysator eine Lipase ist (EC 3.1.1.3 und 3.1.1.79; auch Glycerinester-Hydrolasen, Triacylglycerin-Lipasen, Triglycerid-Lipasen, Triacylglycerin-Acylhydrolasen). Lipasen sind Enzyme, die Lipide wie Triglyceride oder Diglyceride zu Glycerin und freien Fettsäuren umwandeln, indem die Esterbindung katalytisch gespalten wird. Durch den Abbau von Fetten in der Gefäßwand kann der Bildung/dem Wachstum vulnerabler Plaques entgegengewirkt werden. Daher ist der Einsatz von Lipasen erfindungsgemäß bevorzugt.

Bevorzugt ist weiterhin, dass Biokatalysator eine NO-Synthase ist (NOS; EC 1.14.13.39). Weitere Bezeichnungen sind NADPH-Diaphorase Endotheliumderived relaxation factor (EDRF) und der systematische Name lautet L-Arginin, NADPH: Sauerstoffoxidoreduktase. NOS ist ein homodimeres Enzym und setzt unter NADPH/H⁺- und O₂-Verbrauch L-Arginin durch zweimalige Hydroxylierung zu L-Citrullin um, wobei Stickstoffmonoxyd (NO) entsteht. NO ist für viele wichtige Funktionen im Gefäßsystem verantwortlich:
- Vasodilatation (Gefäßweitung),
- Hemmung von gefäßverengenden Einflüssen,
- Hemmung der Thombozytenaggregation an der Gefäßinnenwand,
- Hemmung der Leukozytenaggregation an der Gefäßinnenwand,
- antiproliferative Wirkung,
- Abfangen der Superoxydanionen.

NO ist weiterhin als Signalüberträger in verschiedenen biologischen Prozessen aktiv: es leitet Signale von Nervenzelle zu Nervenzelle weiter, zirkuliert in der Blutbahn und wirkt dort blutdrucksenkend und es ist wichtig für die Immunabwehr. Die zellulären Reaktionen dieses Radikals sind sehr komplex. Es kann extrem schnell durch Zellmembranen bei einer Lebensdauer von weniger als 20 Sekunden diffundieren. Im Blutgefäßsystem spielt NO eine zentrale Rolle. Wird es vermindert freigesetzt, ist ein Risikofaktor für Erkrankungen der Herzkranzgefäße (koronare Herzerkrankung) und für Gefäßverkalkungen (Arteriosklerose) erhöht. Ausreichende NO-Ausschüttung bildet einen antiarteriosklerotischen Schutz, indem es das Wachstum wuchernder Zellen hemmt und ein Anhaften von Blutplättchen und Blutkörperchen an der Gefäßwand verhindert. Hinzu kommt seine vasodilatorische Wirkung, auf der die Anwendung von Nitrospray bei akutem Herzanfall (Angina pectoris) beruht. NO ist ein vom Endothel der Gefäßwand gebildeter Botenstoff, welcher zu einer Relaxation der glatten Muskelzellen führt und ein wichtiger Mediator, welcher vom Endothel als Antwort auf physiologische Stimuli wie Katecholamine (Sympathikus-Stimulation) oder eine Blutflusssteigerung (körperliche Belastung) gebildet wird und zur Vasorelaxation führt. Vom Endothel gebildetes NO wird als endogenes NO bezeichnet. Zur Wirkung von NO siehe auch: R. Sarkar, R.C. Webb, J Vasc Res 1998, 35, 135 - 142, Titel: Does Nitric Oxide Regulate Smooth Muscle Cell Proliferation?; T.F. Lüscher et al., Internist 1997, 38, 411 - 419, Titel: Endotheliale Dysfunktion und Stickstoffmonoxid (NO; Nitric Oxide). Die angesprochenen physiologischen Wirkungen des NO lassen den Einsatz zur Minderung oder Verhinderung der Restenose nach Stentimplantation, insbesondere im Falle einer vorangehenden perkutanten transluminalen koronaren Angioplastie (PTCA), aussichtsreich erscheinen. Aus diesem Grunde ist die kovalente Anbindung einer NO-Synthase besonders bevorzugt.

Denkbar ist auch, die für die Umsetzung notwendigen Edukte an der Implantatsoberfläche bereitzustellen, also im Falle einer Glucoseoxidase insbesondere β-D-Glucose und im Falle einer NO-Synthase insbesondere L-Arginin und NADPH.

Definitionsgemäß werden Biokatalysatoren als immobilisiert bezeichnet, wenn sie auf künstlichem Wege in eine katalytisch aktive, unlösliche Form gebracht wurden. Eine Anbindung erfolgt erfindungsgemäß durch Ausbildung einer kovalenten Bindung zwischen dem Biokatalysator und einer zur Implantatsoberfläche gehörigen reaktiven Funktionalität. Durch die kovalente Bindung kann eine sehr stabile Anbindung bei ausreichend hoher Aktivität der angebundenen Biokatalysatoren erreicht werden. Zur kovalenten Bindung muss die Oberfläche demnach reaktive Gruppen aufweisen, die mit funktionellen Gruppen an der Oberfläche der Biokatalysatoren kovalente Bindungen eingehen können. Geeignete Gruppen in Proteinen sind Carboxy-, Hydroxy- und Sulfid-Gruppen und insbesondere die endständige Amino-Gruppe von Lysin. Die Oberflächen werden direkt oder indirekt (über bi- und multifunktionelle Verbindungen) für die Anbindung der Enzyme aktiviert. Die Methoden unterscheiden sich in Protein-Beladung, Kupplungsausbeute und Einfluss auf die Aktivität der Biokatalysatoren. Durch längere Molekülketten (englisch spacer) kann der Abstand des Biokatalysators von der Implantatsoberfläche vergrößert werden. HydroxyGruppen von Polymeren können mit Bromcyan, N-Hydroxy-succinimidester, Carbonyldiimidazol, Chlortriazin, Thionylchlorid, Divinylsulfon, Chloressigsäure, Epoxiden, Natriumperiodat, Tosyl- und Tresylchlorid aktiviert werden, die Oberfläche von porösen Gläsern und Kieselgelen durch bifunktionelle Silane (z. B. Aminoalkyltriethoxysilan oder Glycidoxypropyltrimethoxysilan u. a.), AminoGruppen am Träger mit Glutardialdehyd, Chlortriazin, Glycidol oder durch Diazotierung, Carboxy-Gruppen durch Carbonyldiimidazol, wasserlösliches Carbodiimid und aktivierte Ester und Sulfid-Gruppen durch Disulfid-Austausch.

Vorzugsweise sind die Biokatalysatoren an Funktionalitäten gebunden, die durch eine vorhergehende Behandlung der Oberfläche des Implantats eingeführt wurden, nämlich
(i) durch Silanisierung der Oberfläche mit Aminosilanen und damit Bereitstellen von Aminfunktionen, oder
(ii) durch Behandlung der Oberfläche mit einer Disäure und damit Bereitstellen von Säurefunktionen.

Die genannten Funktionalisierungen der Oberfläche zeichnen sich durch einen kostengünstigen Zugang zu den notwendigen Edukten, eine bewährte und sichere Verfahrensführung sowie eine mit hohen Ausbeuten realisierbare und reproduzierbare anschließende Anbindung der Biokatalysatoren aus. Vorzugsweise wird in Variante (i) ein Aminosilan ausgewählt aus der Gruppe 3-Aminopropyltrimethoxysilan, 3-(Trimethoxysilylpropyl)-diethylentriamin, N-(2-Aminoethyl)-3-aminopropyltrimethoxysilan, 3-Aminopropyltriethoxysilan oder N-(6-Aminohexyl)-aminopropyltrimethoxysilan eingesetzt. Besonders bevorzugt ist das Aminosilan 3-Aminopropyltriethoxysilan, da sich bei Verwendung dieser Verbindung die größte Aminbeladung auf dem Substrat erzielen ließ. In Variante (ii) wird vorzugsweise Adipinsäure eingesetzt.

Eine Anbindung nach Variante (i) erfolgt vorzugsweise mit Glutaraldehyd oder einem Carbodiimid, und eine Anbindung nach Variante (ii) erfolgt vorzugsweise mit einem Carbodiimid. Die genannten Verfahrensvarianten zeichnen sich durch eine gute Zugänglichkeit der Edukte, besonders einfache und sichere Verfahrensführung und hohen Ausbeuten bei der Kopplung der Biokatalysatoren aus.

Weitere bevorzugte Ausführungsformen der Erfindung lassen sich den nachfolgenden Ausführungsbeispielen entnehmen.

### Ausführungsbeispiel 1 - Enzymanbindung an Glassubstrate

Es wurden Glassubstrate eingesetzt, deren Oberflächen von etwaigen Verunreinigungen befreit und aktiviert wurden. Eine Aktivierung kann durch saure Aufbereitung (Bereitstellung von Hydroxylgruppen an der Oberfläche) oder durch Plasmabeschichtung (Bereitstellung von Hydroxyl- oder Amingruppen an der Oberfläche) erfolgen. Die Aktivierung vereinfacht die Anbindung der nachfolgend eingeführten Funktionalitäten.

Die aktivierten Glassubstrate wurden nach einer ersten Alternative in herkömmlicher Verfahrensweise mit den folgenden Aminosilanen funktionalisiert: 3-Aminopropyltrimethoxysilan, 3-(Trimethoxysilylpropyl)-diethylentriamin, N-(2-Aminoethyl)-3-aminopropyltri-methoxysilan, 3-Aminopropyltriethoxysilan und N-(6-Aminohexyl)-aminopropyltrimethoxysilan. Durch die Funktionalisierung stehen Amingruppen zur kovalenten Anbindung der Enzyme bereit.

### a) Kopplung von Enzymen mit Glutaraldehyd

Das Vorgehen eignet sich zur Anbindung an Aminfunktionalitäten.

Ein aktiviertes Glassubstrat wurde mit einer 2,5 %igen Glutaraldehydlösung (gepuffert mit Phosphatpuffer auf pH 8) für eine Stunde bei Raumtemperatur inkubiert. Nach der herkömmlichen Aufbereitung der Reaktionslösung wurde eine Glucoseoxidase ebenfalls bei Raumtemperatur und in einem gepufferten System bei pH 8 für eine Stunde inkubiert. Anschließend wurde die gebildete Schiffsche Base durch Zusatz eines Äquivalents NaBH₃CN in Wasser reduziert.

### b) Kopplung von Enzymen mit 1-Ethyl-3-(3-dimethylaminopropyl)carbodiimide (EDC)

Das Vorgehen eignet sich zur Anbindung an Amin- und Säurefunktionalitäten.

Ein aktiviertes Glassubstrat wurde mit 1000 U (22mg) einer Glucoseoxidase in 5 ml Puffer und mit 5 mg EDC inkubiert. Nach 2 Stunden Rühren bei Raumtemperatur wurde das Glassubstrat in herkömmlicher Weise aufgearbeitet.

### Ausführungsbeispiel 2 - Enzymanbindung an einen Stent

Ein mit Siliziumcarbid beschichteter herkömmlicher Stent aus medizinischem Stahl wurde analog der Vorgehensweise aus Beispiel 1 mit einem Aminosilan zur Einbringung einer Aminfunktion silanisiert. Es wurden (i) eine Glucoseoxidase und (ii) eine NO-Synthase mit Hilfe des Carbodiimids EDC kovalent angebunden (analog den Vorschriften aus Beispiel 1).

Über eine spektralphotometrische Bestimmungsmethode zur Erfassung eines Wasserstoffperoxidgehaltes (*o*-Dianisidin-Assay) konnte eine signifikante Enzymaktivität der Glucoseoxidase bestätigt werden.

Über eine spektralphotometrische Bestimmungsmethode zur Erfassung eines Nitritgehaltes (Grieß-Ilosvay-Reaktion) konnte eine signifikante Enzymaktivität der NO-Synthase bestätigt werden.

## Patentansprüche

1. Medizinisches Implantat mit an seiner Oberfläche durch kovalente Anbindung immobilisierten Biokatalysatoren, **dadurch gekennzeichnet, dass** der Biokatalysator eine Peroxidase ist.

2. Medizinisches Implantat nach Anspruch 1, **dadurch gekennzeichnet, dass** das Implantat ein Stent ist.

3. Medizinisches Implantat nach Anspruch 1 oder 2, **dadurch gekennzeichnet, dass** die Peroxidase eine Katalase ist.

4. Medizinisches Implantat nach Anspruch 1, **dadurch gekennzeichnet, dass** die Biokatalysatoren an Funktionalitäten gebunden sind, die durch Silanisierung der Oberfläche mit Aminosilanen eingeführt wurden.

5. Medizinisches Implantat nach Anspruch 1, **dadurch gekennzeichnet, dass** die Biokatalysatoren an Funktionalitäten gebunden sind, die durch Behandlung der Oberfläche mit einer Disäure eingeführt wurden.
